# EUROPEAN PATENT SPECIFICATION

(11) **EP 1 957 450 B1**
(45) Date of publication and mention of the grant of the patent: **24.06.2009**
(21) Application number: 06819656.7
(22) Date of filing: 22.11.2006
(51) Int. Cl.: C07D 207/08, C07D 207/09, A61K 31/40

(54) **SEROTONIN TRANSPORTER (SERT) INHIBITORS**
INHIBITOREN DES SEROTONINTRANSPORTERS (SERT)
INHIBITEURS DU TRANSPORTEUR DE SEROTONINE (SERT)

(30) Priority: 01.12.2005 EP 05111565
(43) Date of publication of application: 20.08.2008
(73) Proprietor: F.HOFFMANN-LA ROCHE AG, 4070 Basel (CH)
(72) Inventor: GRUNDSCHOBER, Christophe, CH-4118 Rodersdorf (CH); HOFFMANN, Torsten, 79576 Weil am Rhein (DE); KOBLET, Andreas, CH-4103 Bottmingen (CH); SCHNIDER, Patrick, CH-4103 Bottmingen (CH)
(74) Representative: Poppe, Regina
(86) International application number: PCT/EP2006/068740
(87) International publication number: WO 2007/063009

(56) References cited:
- WO-A-03/015784
- WO-A1-03/042173
- WO-A2-20/05032464
- WO-A2-20/06014665
- RYCKMANS, THOMAS ET AL: "Dual NK1 antagonists-serotonin reuptake inhibitors as potential antidepressants. Part 2: SAR and activity of benzyloxyphenethyl piperazine derivatives" BIOORGANIC & MEDICINAL CHEMISTRY LETTERS, vol. 12, no. 21, 2002, pages 3195-3198, XP002418848 cited in the application
- RYCKMANS, THOMAS ET AL: "First dual NK1 antagonists-serotonin reuptake inhibitors: synthesis and SAR of a new class of potential antidepressants" BIOORGANIC & MEDICINAL CHEMISTRY LETTERS, vol. 12, no. 2, 2002, pages 261-264, XP002418849 cited in the application

## Description

The present invention relates to cis-derivatives of formula 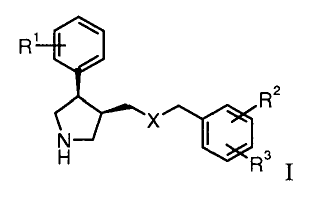 wherein
- R¹: is hydrogen, halogen or lower alkyl;
- R²/R³: are independently from each other hydrogen, halogen, CN, lower alkyl, lower alkyl substituted by halogen, lower alkoxy or lower alkoxy substituted by halogen;
- X: is -O- or -N(R)-;
- R: is hydrogen or lower alkyl;
and to pharmaceutically acceptable acid addition salts thereof.

Encompassed by the present formula I are compounds of formulas

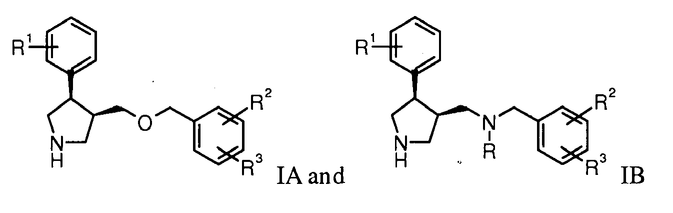

The compounds of formula I are good inhibitors of the serotonin transporter (SERT inhibitors). By virtue of their efficacy as SERT inhibitors, the compounds in the present invention are particularly useful for the treatment of CNS disorders and psychotic disorders, in particular in the treatment or prevention of depressive states and/or in the treatment of anxiety.
SERT Inhibitors including the selective serotonin transporter inhibitors, also called selective serotonin reuptake inhibitors (SSRI's), have become the most frequently prescribed antidepressant drugs. They are believed to exert their effect by increasing extracellular 5-HT levels in the serotoninergic terminal fields such as the hippocampus and prefrontal cortex. However, approximately 30 % of patients appear to be resistant to SSRI treatment. In addition, those patients who do benefit from SSRI treatment often exhibit various side-effects which include sexual dysfunction, gastrointestinal distress, insomnia and in some cases anxiogenesis due to their indirect activation (through elevation of 5-HT levels) of all 5-HT receptors. Furthermore, a common problem in current antidepressant therapies is their slow onset of action, since a delay of about 4 weeks is normally observed between the beginning of the treatment and alleviation of the symptoms. The delay appears to parallel the progressive desensitization of somatodendritic SHT_{1A} receptors, increasing serotoninergic function, thus allowing alleviation of depressive symptoms.
Therefore, the object of the present invention was to find compounds which have SERT inhibitory activity with an additional beneficial effect on the onset of action and which allow major improvements for SSRI-resistant patients, e.g. with a reduced anxiogenic or even anxiolytic profile.
It has surprisingly been found that present compounds of formula I have a good activity as SERT inhibitors and that they are concomitantly active as NK-1 receptor antagonists. NK-1 antagonists are believed to indirectly modulate 5-HT function via noradrenergic pathways and have been shown to attenuate presynaptic SHT_{1A} receptor function (Bioorg. Med. Chem., Lett. 12, (2002), 261-264*)*.
Thus, combination of serotonin uptake inhibition with NK-1 antagonism may lead to compounds with an improved onset of action and a better efficacy during the treatment of depressive/anxiolytic states.
The compounds of the present invention combine serotonin transporter inhibition and NK-1 antagonism.

Recent reports have indicated that the combination of a SSRI and a NK-1 antagonist produces beneficial responses in animal models of anxiety and depression such as guinea-pig pup maternal separation vocalization, with relatively reduced doses (WO98/47514*; .* Bioorg. Med. Chem. Lett. 12(2), 261-264 (2002*) and* Bioorg. Med. Chem. Lett. 12(21), 3195-3198 (2002*).*

This suggests that by adopting a dual approach which is mechanistically dissimilar, a synergism between the two modes of action may occur, enabling enhanced responses. This may not only be beneficial in patients resistant to treatment with SSRI alone but also in improving the rapidity of onset of therapeutic action. A drug with a dual mode of action potentially allows for a reduction in dosing and therefore a decreased risk of side effects as compared to a combination of two drugs. In the patent literature the combined NK-1/SSRI approach has also been proposed as a potential treatment for obesity *(*WO98/47514*)*.

WO2005/032464 describes trans-phenyl pyrrolidine ethers which are tachykinin receptor antagonists. All described examples in this application have a substitution on the N-atom of the pyrrolidine ring, which seems to be necessary for a selective tachykinin receptor antagonistic activity. Now it has surprisingly been found that a small group of cis-derivatives of pyrrolidine derivatives of the present formula I, which are not substituted on the N-atom of the pyrrolidine ring, are SERT inhibitors and simultaneously, they have a good activity as NK-1 receptor antagonists. A drug with a dual mode of action combines the advantages of both receptor sites, and the dosage of the drug may therefore be reduced, thus leading to a decreased risk of side effects as compared to a combination of two drugs.

It has been shown that trans-derivatives do not have a dual activity or they have a very low dual activity, and therefore they cannot share the advantages as mentioned above. In the table below are shown NK-1 and SERT activities of compounds of present cis-derivatives of formula I, compared with structure-related trans-derivatives of formulas II, III and IV, partially encompassed by WO2005/032464:

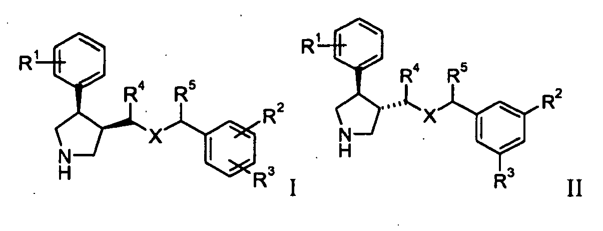

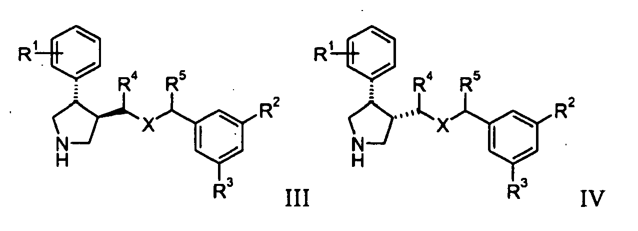

| R¹ | R² | R³ | R⁴ | R⁵ | X | pKi hSERT | pKi hNK1 | Example |
|---|---|---|---|---|---|---|---|---|
| 4-F | 3-CF₃ | 5-CF₃ | H | H | -O- | 8.56 | 6.53 | 1 of I |
| 3-F | 3-CF₃ | 5-CF₃ | H | H | -O- | 8.23 | 7.33 | 2 of I |
| 2-F | 3-CF₃ | 5-CF₃ | H | H | -O- | 8.46 | 7.30 | 3 of I |
| 4-Cl | 3-CF₃ | 5-CF₃ | H | H | -O- | 8.35 | 7.27 | 4 of I |
| 3-Cl | 3-CF₃ | 5-CF₃ | H | H | -O- | 8.09 | 7.50 | 5 of I |
| 2-Cl | 3-CF₃ | 5-CF₃ | H | H | -O- | 7.99 | 7.36 | 6 of I |
| 2-CH₃ | 3-CF₃ | 5-CF₃ | H | H | -O- | 8.12 | 7.54 | 7 of I |
| H | 3-CF₃ | 5-CF₃ | H | H | -O- | 8.31 | 7.36 | 8 of I |
| H | 3-Br | 5-Br | H | H | -O- | 8.23 | 7.38 | 9 of I |
| H | 3-CH₃ | 5-CH₃ | H | H | -O- | 8.34 | 6.59 | 10 of I |
| H | 3-CF₃ | 5-F | H | H | -O- | 8.24 | 6.65 | 11 of I |
| H | 3-CF₃ | H | H | H | -O- | 7.96 | 6.17 | 12 of I |
| H | 3-OCF₃ | H | H | H | -O- | 7.86 | 6.12 | 13 of I |
| H | 3-OCH₃ | 5-OCH₃ | H | H | -O- | 7.69 | 6.13 | 14 of I |
| H | 2-CF₃ | H | H | H | -O- | 7.15 | 6.19 | 15 of I |
| H | 3-CF₃ | 5-CF₃ | H | H | NH | 7.64 | 7.58 | 16 of I |
| H | 3-CF₃ | 5-CF₃ | H | H | N(CH₃) | 8.09 | 7.50 | 17 of I |
| 4-F | CF₃ | CF₃ | H | H | -O- | 6.93 | 8.12 | 1 of II |
| 4-F | CF₃ | CF₃ | H | 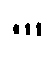 CH₃ | -O- | 6.90 | 8.74 | 2 of II |
| 4-F | CF₃ | CF₃ | H | 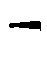 CH₃ | -O- | 6.39 | 8.15 | 3 of II |
| 2-CH₃ | CF₃ | CF₃ | H | 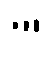 CH₃ | -O- | 5.82 | 8.38 | 4 of II |
| 2-CH₃ | CF₃ | CF₃ | H | 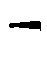 CH₃ | -O- | 6.09 | 7.86 | 5 of II |
| 2-CH₃ | CF₃ | CF₃ | H | H | -O- | 6.28 | 7.90 | 6 of II |
| 3-Cl | CF₃ | CF₃ | H | H | -O- | 6.49 | 7.57 | 7 of II |
| 4-F | CF₃ | CF₃ | H | H | -O- | 6.35 | 7.90 | 1 of III |
| 4-F | CF₃ | CF₃ | H | -CH₃ | -O- | 5.66 | 7.32 | 2 of III |
| 4-F | CF₃ | CF₃ | H | 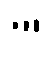 CH₃ | -O- | 5.82 | 8.16 | 3 of III |
| 2-CH₃ | CF₃ | CF₃ | H | H | -O- | 6.64 | 8.43 | 4 of III |
| 2-CH₃ | CF₃ | CF₃ | H | 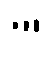 CH₃ | -O- | 6.11 | 8.90 | 5 of III |
| 2-CH₃ | CF₃ | CF₃ | H | 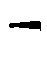 CH₃ | -O- | 6.42 | 8.14 | 6 of III |
| 3-Cl | CF₃ | CF₃ | H | H | -O- | 6.13 | 7.05 | 7 of III |
| 4-Cl | CF₃ | CF₃ | H | H | -O- | 6.32 | 7.29 | 8 of III |
| 2-F | CF₃ | CF₃ | H | H | -O- | 6.96 | 8.10 | 9 of III |
| 4-F | CF₃ | CF₃ | H | H | -O- | 6.79 | 6.36 | 1 of IV |
| 3-F | CF₃ | CF₃ | H | H | -O- | 6.58 | 7.20 | 2 of IV |
| 2-CH₃ | CF₃ | CF₃ | H | 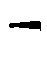 CH₃ | -O- | 6.54 | 6.20 | 3 of IV |
| 2-CH₃ | CF₃ | CF₃ | H | 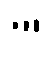 CH₃ | -O- | 6.35 | 6.08 | 4 of IV |

Related compounds of formulas II and III have a high selectivity to the NK-1 receptor (not desired in the present case) and compounds of formula IV show an activity on both receptors on an unexpectedly low level.
Furthermore, it has been found that a substitution on the N-atom in the pyrrolidine-ring leads to a decrease of the SERT activity, as shown below: 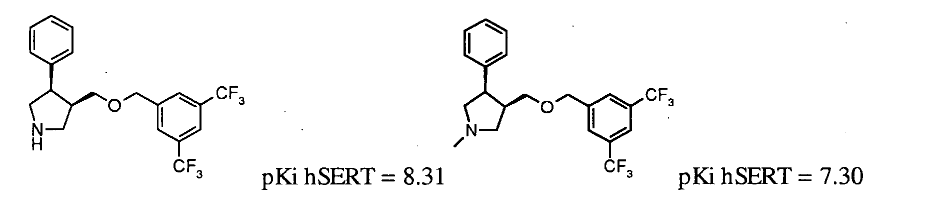 The data has been generated in accordance with the following assays:
hSERT SPA binding assay
HEK-293 cells stably expressing recombinant human SERT are maintained with DMEM high glucose with 10 % FBS, 300 µg/ml G418 and 2 mM L-Glutamine and incubated at 37 °C with 5 % CO₂. Cells are released from culture flasks using PBS for 1-2min. The cells are subsequently centrifuged at 1000 g's for 5 min and resuspended in PBS prior to being used in the membrane preparation.
Cells are homogenized using a Polytron in 50 mM Tris (pH 7.4). Centrifuged at 48,000 xg for 15 min, and the pellet resuspended in fresh buffer. After a second centrifugation, the pellet is re-homogenized and resuspended in fresh buffer. Typically, membrane portions are aliquoted in 3mg/ml (w:v). and stored at -80 °C.

A serial dilution of test compounds in 50 mM Tris-HCl, 120 mM NaCl, KCl 5 mM (pH 7.4) is made in a white Optiplate (Packard) (100µl/well) and the radioligand ³[H] Citalopram (Specific activity: 60-86 Ci/mmol, Final concentration: 1 nM) is added at 50 µl/well. Membrane and beads are prepared to a ratio of 5 µg:0.6 mg, with 0.6 mg PVT-WGA Amersham beads (Cat# RPQ0282V) added per well. 50 µl of the membrane/bead mixture is added to the assay plate for a final volume of 200µl. The mixtures are allowed to stand at room temperature for one hour, and are then counted on a Packard TopCount.

The % inhibition is calculated for each compound tested (with 100 % binding being the value obtained with the incubation of membrane/beads and radioligand in buffer without compound minus the non-specific binding measured in presence of 10µM Fluoxetine). The concentration producing 50 % inhibition (IC₅₀) is determined using an iterative non-linear curve fitting technique. The inhibition dissociation constant (Ki) of each compound is determined according to the method of Cheng-Prusoff.
hNK-1 binding assay

The affinity of test compounds for the NK-1 receptor was evaluated at human NK-1 receptors in CHO cells transfected with the human NK-1 receptor using the Semliki virus expression system and radiolabelled with [³H] substance P (final concentration 0.6 nM). Binding assays were performed in HEPES buffer (50 mM, pH 7.4) containing BSA (0.04 %) leupeptin (8 µg / ml), MnCl₂ (3mM) and phosphoramidon (2 µM). Binding assays consisted of 250 µl of membrane suspension (1.25x10⁵ cells / assay tube), 125 µl of buffer of displacing agent and 125 µl of [³H]substance P. Displacement curves were determined with at least ten concentrations of the compound. The assay tubes were incubated for 60 min at room temperature after which time the tube contents were rapidly filtered under vacuum through GF/C filters presoaked for 60 min with PEI (0.3 %) with 2 x 2 ml washes of HEPES buffer (50 mM, pH 7.4). The radioactivity retained on the filters was measured by scintillation counting. All assays were performed in duplicate in at least 2 separate experiments.

The inhibition dissociation constant (Ki) of each compound for NK1 is determined as described above for hSERT.

### References

- Froger, N., Gardier, A.M., Moratalla, R., et al., 5-HT1A autoreceptor adaptive changes in substance P (Neurokinin 1) receptor knock-out mice mimic antidepressant-induced desensitization. J. Neuroscience, 21(20), 8188-8197 (2001).
- Kramer M.S., Cutler, N., Feighner, J. et al., Distinct Mechanism of antidepressant activity by blockade of central substance P receptors. Science 281, 1640-1645 (1998).
- Millan, M.J., Lejeune, F., de Nanteuil, G. and Gobert, A., A selective blockade of neurokinin NK1 receptors facilitates the activity of adrenergic pathways projecting to the frontal cortex and dorsal hippocampus in rats. J. Neurochem., 76, 1949-1954 (2001).
- Rupniak, N.M.J. New Insights into the antidepressant actions of substance P (NK1 receptor) antagonists. N.M.J Can. J. Physiol. Pharmacol. 80, 489-494 (2002).
- Rupniak, N.M.J. Elucidating the antidepressant actions of substance P (NK1 receptor) antagonists. Current Opinion in Investigational Drugs, 3(2), 257-261 (2002).
- Ryckmans, T., Balancon, L., Berton, O., et al., First dual NK1 antagonists-serotonin reuptake inhibitors: synthesis and SAR of a new class of potential antidepressants. Bioorg. Med. Chem. Lett. 12(2), 261-264 (2002).
- Ryckmans, T., Berton, O., Grimee, R., et al., Dual NK1 Antagonists-serotonin reuptake inhibitors as potential antidepressants. Part 2 :SAR and activity of benzyloxyphenethyl piperazine derivatives. Bioorg. Med. Chem. Lett. 12(21), 3195-3198 (2002).
- WO98/47514 A1. Use of an NK1 receptor antagonist and an SSRI for treating obesity.
- WO03/015784 A1. 2-Substituted 1-arylpiperazines as tachykinin antagonists and/or serotonin reuptake inhibitors.

Objects of the present invention are the compounds of formula I per se, the use of compounds of formula I and their pharmaceutically acceptable salts for the manufacture of medicaments for the treatment of diseases related to activation of Serotonin Transporter (SERT), such as for the treatment of depression and anxiety, their manufacture, medicaments based on a compound in accordance with the invention and their production as well as the use of compounds of formula I in the control or prevention of illnesses such as depression and anxiety.

The invention includes all cis-derivatives and their pharmaceutically active salts.

As used herein, the term "lower alkyl" denotes a saturated straight- or branched-chain group containing from 1 to 7 carbon atoms, for example, methyl, ethyl, propyl, isopropyl, n-butyl, i-butyl, 2-butyl, t-butyl and the like. Preferred alkyl groups are groups with 1 - 4 carbon atoms.

The term "halogen" denotes chlorine, iodine, fluorine and bromine.

The term "lower alkyl substituted by halogen" denotes a saturated straight- or branched-chain group containing from 1 to 7 carbon atoms as described for "lower alkyl", wherein at least one hydrogen atom is replaced by a halogen atom, for example CF₃, CHF₂ or CH₂F.

The term " lower alkoxy" denotes a residue O-R, wherein R is a saturated straight- or branched-chain group containing from 1 to 7 carbon atoms as described for "lower alkyl".

The term " lower alkoxy substituted by halogen" denotes a residue O-R, wherein R is a saturated straight- or branched-chain group containing from 1 to 7 carbon atoms as described for lower alkyl, wherein at least one hydrogen atom is replaced by a halogen atom, for example OCF₃, OCHF₂ or OCH₂F.

The term "pharmaceutically acceptable acid addition salts" embraces salts with inorganic and organic acids, such as hydrochloric acid, nitric acid, sulfuric acid, phosphoric acid, citric acid, formic acid, fumaric acid, maleic acid, acetic acid, succinic acid, tartaric acid, methane-sulfonic acid, p-toluenesulfonic acid and the like.

Preferred compounds of formula I are those of formula I-A, for example the following compounds:
(+)-(3R,4R)-3-(3,5-bis-trifluoromethyl-benzyloxymethyl)-4-(4-fluoro-phenyl)-pyrrolidine,
(+)-(3R,4R)-3-(3,5-bis-trifluoromethyl-benzyloxymethyl)-4-(3-fluoro-phenyl)-pyrrolidine,
(+)-(3R,4R)-3-(3,5-bis-trifluoromethyl-benzyloxymethyl)-4-(2-fluoro-phenyl)-pyrrolidine,
(+)-(3R,4R)-3-(3,5-bis-trifluoromethyl-benzyloxymethyl)-4-(4-chloro-phenyl)-pyrrolidine,
(+)-(3R,4R)-3-(3,5-bis-trifluoromethyl-benzyloxymethyl)-4-(3-chloro-phenyl)-pyrrolidine,
(+)-(3R,4R)-3-(3,5-bis-trifluoromethyl-benzyloxymethyl)-4-(2-chloro-phenyl)-pyrrolidine,
(+)-(3R,4R)-3-(3,5-bis-trifluoromethyl-benzyloxymethyl)-4-o-tolyl-pyrrolidine,
(+)-(3R,4R)-3-(3,5-bis-trifluoromethyl-benzyloxymethyl)-4-phenyl-pyrrolidine,
(+)-(3R,4R)-3-(3,5-dibromo-benzyloxymethyl)-4-phenyl-pyrrolidine,
(+)-(3R,4R)-3-(3,5-dimethyl-benzyloxymethyl)-4-phenyl-pyrrolidine,
(+)-(3R,4R)-3-(3-fluoro-5-trifluoromethyl-benzyloxymethyl)-4-phenyl-pyrrolidine,
(+)-(3R,4R)-3-phenyl-4-(3-trifluoromethyl-benzyloxymethyl)-pyrrolidine,
(+)-(3R,4R)-3-phenyl-4-(3-trifluoromethoxy-benzyloxymethyl)-pyrrolidine,
(+)-(3R,4R)-3-(3,5-dimethoxy-benzyloxymethyl)-4-phenyl-pyrrolidine,
(+)-(3R,4R)-3-phenyl-4-(2-trifluoromethyl-benzyloxymethyl)-pyrrolidine.

Preferred compounds of formula I are further those of formula I-B, for example the following compounds
(+)-(3,5-bis-trifluoromethyl-benzyl)-((3R,4R)-4-phenyl-pyrrolidin-3-ylmethyl)-amine dihydrochloride or
(3,5-bis-trifluoromethyl-benzyl)-methyl-((3R,4R)-4-phenyl-pyrrolidin-3-ylmethyl)-amine dihydrochloride.

The novel cis-derivatives of formula I and their pharmaceutically acceptable salts can be prepared by methods known in the art, for example by processes described below, which processes comprise
a) reacting a compound of formula 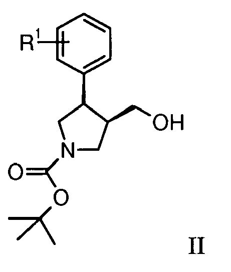 with sodium hydride and a compound of formula 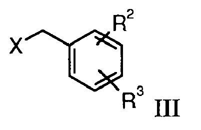 followed by treatement with acid such as HCl or trifluoroacetic acid to give a compound of formula 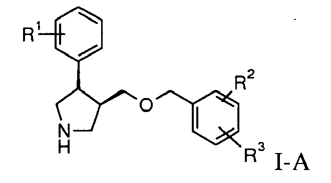 wherein X is Cl, Br or I and R¹ to R³ are as described above, or
b) reacting a compound of formula 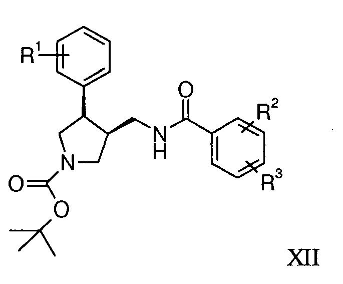 with a THF solution of borane dimethylsulfide complex in toluene and then with acid such as HCl or trifluoroacetic acid to a compound of formula 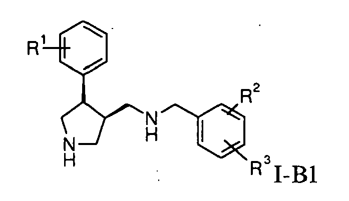 wherein the substituents are as described above, or
c) alkylating a compound of formula 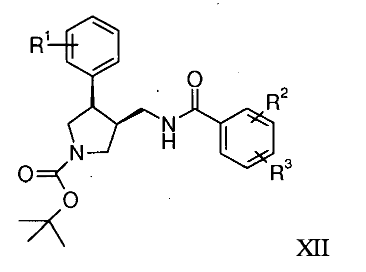 with an alkylating agent, such as methyliodide to a compound of formula 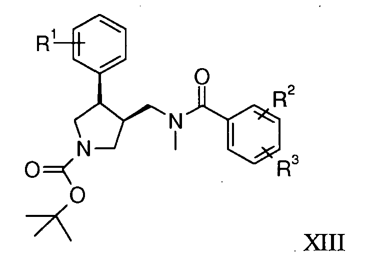 which is consecutively reacted with a THF solution of borane dimethylsulfide complex in toluene and then with acid such as HCl or trifluoroacetic acid to a compound of formula 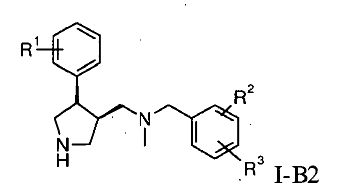
wherein the substituents are as described above, or
if desired, converting the compounds obtained into pharmaceutically acceptable acid addition salts.

Schemes I and 2 show the preparation of compounds of formula I in more detail. The starting material used in schemes 1 and 2 are known compounds or may be prepared by methods known in the art.

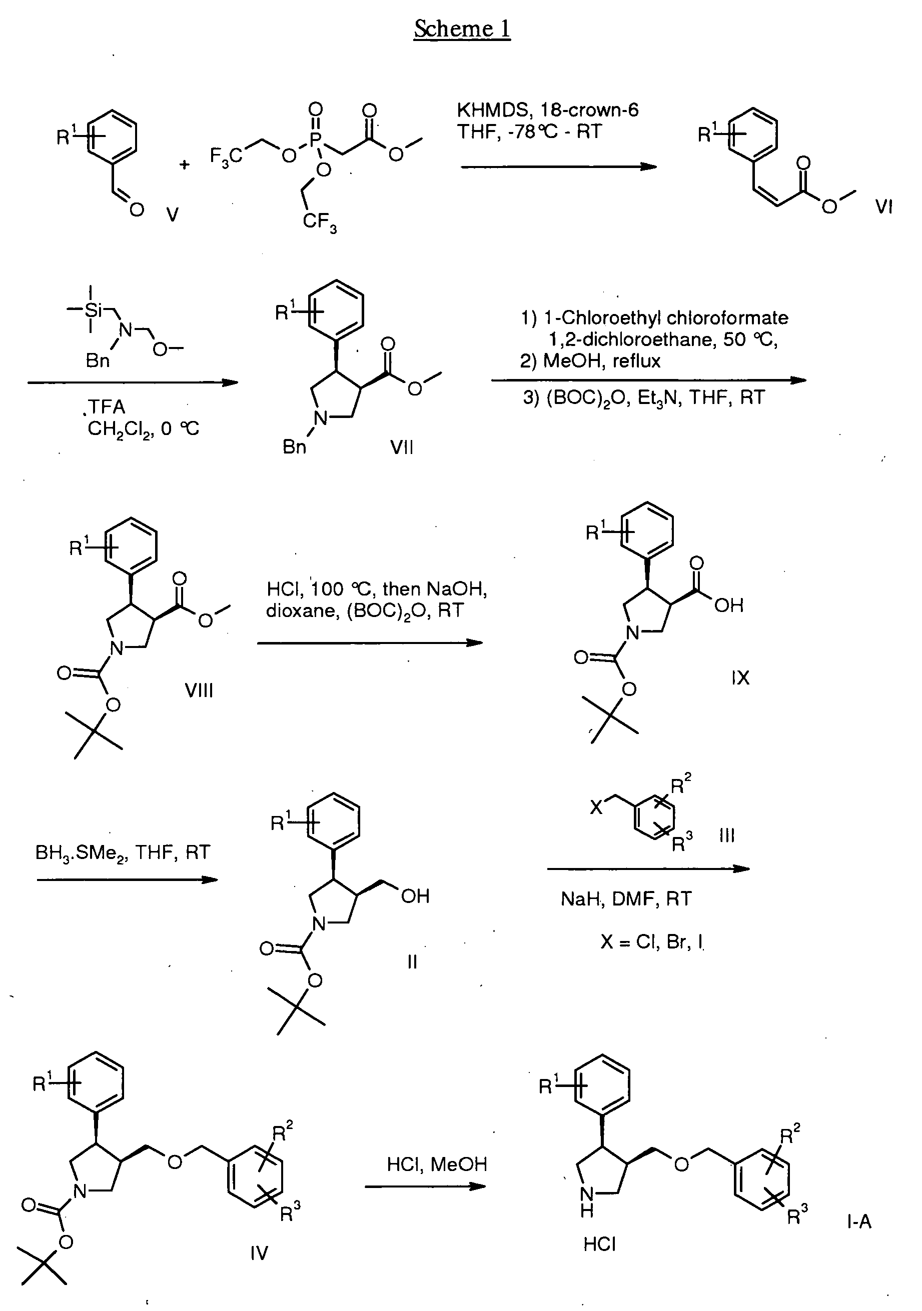

In accordance with scheme 1, compounds of formula I-A may be prepared as follows: To a solution of bis(2,2,2-trifluoroethyl) (methoxycarbonylmethyl)-phosphonate and 18-crown-6 THF is added potassium hexamethyldisilazide solution at -78 °. After 5 min a compound of formula V is added. The reaction mixture is stirred at -78 °C for about 20 min and consequently allowed to warm to room temperature over a period of about 1 h, followed by quenching with a saturated aqueous solution of ammonium chloride and by extraction in conventional manner. The obtained compound of formula VI is dissolved in dichloromethane and consecutively treated with N-(methoxymethyl)-N-(trimethylsilylmethyl)benzylamine (96%, Aldrich) and a solution of dichloromethane and trifluoroacetic acid is added at 0 °C. Another portion of N-(methoxymethyl)-N-(trimethylsilylmethyl)benzylamine (96%, Aldrich) is added after one hour if necessary to drive conversion to completion. The reaction mixture is diluted with dichloromethane and washed with saturated sodium carbonate solution. After concentration and purification a compound of formula VII is obtained. A solution of a compound of formula VII and 1-chloroethyl chloroformate in 1,2-dichloroethane is heated at about 50 °C over night. After cooling to room temperature the solvent is evaporated. The residue is redissolved in MeOH and the resulting solution is heated at reflux for 30 min. The reaction mixture is concentrated in vacuo. The residual hydrochloride salt is redissolved in THF. The mixture is cooled to 0 °C and treated with triethylamine and a solution of di-tert.-butyl dicarbonate in THF. After stirring for about 90 min the reaction mixture is worked up in conventional manner to obtain a compound of formula VIII. Then a mixture of a compound of formula VIII and hydrochloric acid solution is heated at reflux for about 4 h. The reaction mixture is cooled to 0 °C and basified by the addition of sodium hydroxide. Dilution with dioxane is followed by addition of a solution of di-tert.-butyl dicarbonate in 1,4-dioxane. The reaction mixture is concentrated and purified in conventional manner to obtain a compound of formula IX.

To a solution of such acid (IX) in THF is added a borane dimethylsulfide complex solution in THF at 0 °C. The mixture is stirred for about 15 min at 0 °C and then for 5 h at room temperature. After cooling to 0 °C the reaction is quenched by the addition of methanol. Stirring is continued until no evolution of gas is observed any more. The reaction mixture is concentrated in vacuo and purified to obtain a compound of formula II.

Furthermore, to a solution of a compound of formula II in DMF is added sodium hydride at 0 °C. The reaction mixture is allowed to warm to room temperature. After about 1 h a compound of formula III is added. Stirring at room temperature for 2.5 h is followed by quenching with water, extraction and purification. Then a solution of a compound of formula IV in hydrochloric acid and methanol is stirred for 30' at about 50 °C. The reaction mixture is concentrated to dryness to give a compound of formula I-A.

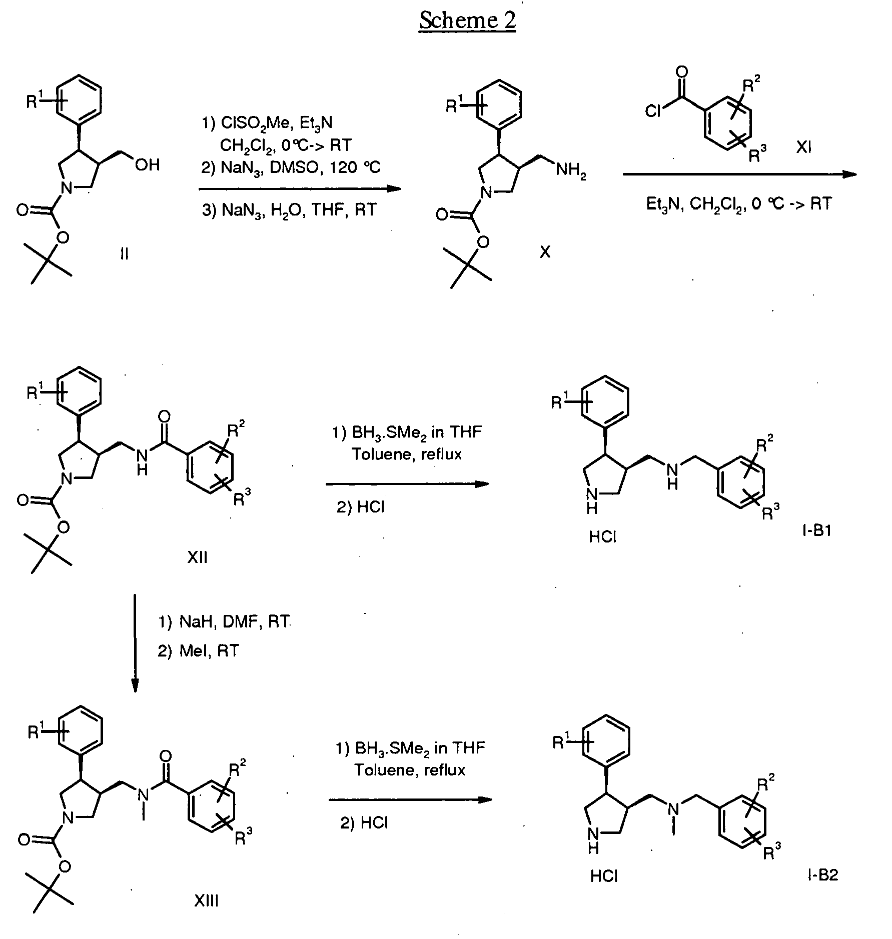

Compounds of formulas I-B1 and I-B2 maybe prepared as follows:

To a solution of a compound of formula II and triethylamine in dichloromethane is added dropwise at 0 °C methanesulfonyl chloride. After completed addition the reaction mixture is allowed to warm to room temperature during 30 minutes. Quenching with water is followed by extraction. The obtained crude product is used in the next step without purification. A mixture of this product and sodium azide in dimethylsulfoxide is heated at about 120 °C for 5 h. The mixture is cooled to room temperature, diluted with a aqueous solution of sodium carbonate and extracted with methyl tert.-butyl ether. The combined organic layers are washed and dried to give the corresponding crude 3-azidomethyl-4-phenyl-pyrrolidine-1-carboxylic acid tert-butyl ester. This compound is dissolved in THF and treated with triphenylphosphine and water. After stirring at room temperature for about 20 h the reaction mixture is diluted with methyl tert.-butyl ether, washed and purified to give a compound of formula X.

Then to a solution of a compound of formula X and triethylamine in dichloromethane is added a compound of formula XI at 0 °C. After completed addition the reaction mixture is allowed to warm to room temperature and stirred for one hour. Quenching with water is followed by basification with sodium hydroxide solution, extraction and purification. It is obtained a compound of formula XII.

Then a mixture of a compound of formula XII and of a THF solution of borane dimethylsulfide complex in toluene is heated at reflux for about 20 h. After addition of another portion of a THF solution of borane dimethylsulfide complex the mixture is heated at reflux for one more hour. After cooling to room temperature methanol is added, and the mixture is heated to reflux for about 30 minutes to give the free base of the compound of formula I-B1.

A compound of formula I-B2 may be obtained as follows:

To a solution of a compound of formula XII in DMF is added sodium hydride at room temperature. After 30 minutes methyl iodide is added, and stirring is continued for about 2 h. Quenching with water is followed by extraction und purification to give a compoundof formula XIII.

A mixture of a compound of formula XIII and of a THF solution of borane dimethylsulide complex in toluene is heated at reflux for about 20 h. After cooling to room temperature an aqueous hydrochloric acid solution is added, and the mixture is heated to reflux for about 30 minutes. The reaction mixture is cooled to room temperature, diluted with aqueous hydrochloric acid solution and washed with methyl tert.-butyl ether. The combined organic layers are extracted with aqueous hydrochloric acid solution. The combined aqueous layers are basified with aqueous sodium hydroxide solution and extracted with dichloromethane. The combined organic extracts are dried and concentrated in vacuo. It is obtained a compound of formula I-B2.

The following examples describe the preparation of compounds of formula I in more detail.

### Intermediate A

### (+)-3-(4-Fluoro-phenyl)-4-hydroxymethyl-pyrrolidine-1-carboxylic acid tert-butyl ester

### a) (Z)-3-(4-Fluoro-nhenyl)-acrylic acid methyl ester

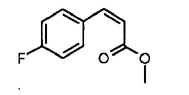

To a solution of 3.45 g (10.9 mmol) bis(2,2,2-trifluoroethyl) (methoxycarbonylmethyl)-phosphonate and 7.18 g (27.2 mmol) 18-crown-6 in 220 ml THF were added 11.9 ml (10.9 mmol) potassium hexamethyldisilazide solution (0.91 M in THF) at -78 °. After 5 min 1.5 g (10.9 mmol) 4-fluorobenzaldehyde were added. The reaction mixture was stirred at -78 °C for 20 min and consequently allowed to warm to room temperature over a period of 1 h. Quenching with a saturated aqueous solution of ammonium chloride was followed by extraction with methyl tert.-butyl ether. The combined organic layers were washed with brine, dried over sodium sulfate and concentrated in vacuo. The crude product was purified by flash column chromatography (silica gel, heptane / ethyl acetate) to give 1.56 g (80%) of the title compound as a slightly yellow oil.

MS m/e (%): 180 (M⁺, 68)

### b) (3RS, 4RS)-1-Benzyl-4-(2-fluoro-phenyl)-pyrrolidine-3-carboxylic acid methyl ester

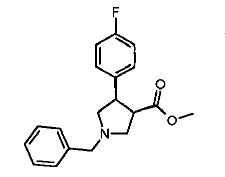

To a solution of 1.56 g (8.66 mmol) (Z)-3-(4-fluoro-phenyl)-acrylic acid methyl ester and 2.26 g (9.14 mmol) N-(methoxymethyl)-N-(trimethylsilylmethyl)benzylamine (96%, Aldrich) in 40 ml dichloromethane were added 0.066 ml (0.87 mmol) trifluoroacetic acid as a solution in 1 ml dichloromethane at 0 °C. Another portion of 1.03 g (4.16 mmol) N-(methoxymethyl)-N-(trimethylsilylmethyl)benzylamine (96%, Aldrich) was added after one hour. After complete consumption of (Z)-3-(4-fluoro-phenyl)-acrylic acid methyl ester the reaction mixture was diluted with dichloromethane and washed with saturated sodium carbonate solution. The aqueous layer was extracted with two portions of dichloromethane. The combined organic layers were washed with brine, dried over sodium sulfate and concentrated in vacuo. The crude product was purified by flash column chromatography (silica gel, heptane / ethyl acetate) to give 2.36 g (87%) of the title compound as a slightly yellow oil.

MS m/e (%): 314 (M+H⁺, 100)

### c) (+)-4-(4-Fluoro-phenyl)-pyrrolidine-1,3-dicarboxylic acid 1-tert-butyl ester 3-methyl ester

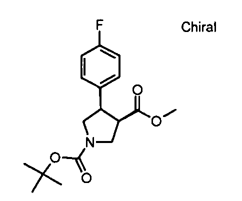

A solution of 7.65 g (24.4 mmol) (3RS, 4RS)-1-benzyl-4-(2-fluoro-phenyl)-pyrrolidine-3-carboxylic acid methyl ester and 3.2 ml (29 mmol) 1-chloroethyl chloroformate in 80 ml 1,2-dichloroethane was heated at 50 °C over night. After cooling to room temperature the solvent was evaporated. The residue was redissolved in 80 ml MeOH and the resulting solution was heated at reflux for 30 min. The reaction mixture was concentrated in vacuo. The residual hydrochloride salt was redissolved in 50 ml THF. The mixture was cooled to 0 °C and treated with 3.4 ml (24 mmol) triethylamine and a solution of 6.4 g (29 mmol) di-tert.-butyl dicarbonate in 30 ml THF. After stirring for 90 min the reaction mixture was diluted with methyl tert.-butyl ether and water. The pH of the aqueous layer was adjusted to 2 by the addition of 2 M aqueous hydrochloric acid solution. The layers were separated and the aqueous layer was extracted with two more portions of methyl tert.-butyl ether. The combined organic layers were dried over sodium sulfate, concentrated in vacuo and purified by flash chromatography to give 5.82 g (74%) of (3RS, 4RS)-4-(4-fluoro-phenyl)-pyrrolidine-1,3-dicarboxylic acid 1-tert-butyl ester 3-methyl ester as a slightly yellow oil.

The enantiomers were separated by chiral HPLC (Chiralpak AD; heptane / EtOH 95 : 5) to give 2.36 g of the title compound as slightly yellow oil.
MS m/e (%): 324 (M+H⁺, 11), 346 (M+Na⁺, 29)
[α]_{D} = +54.98 (c = 0.422, CHCl₃)

### d) (+)-4-(4-Fluoro-phenyl)-pyrrolidine-1,3-dicarboxylic acid 1-tert-butyl ester

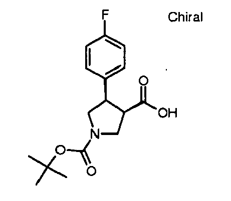

A mixture of 1.79 g (5.54 mmol) (+)-4-(4-fluoro-phenyl)-pyrrolidine-1,3-dicarboxylic acid 1-tert-butyl ester 3-methyl ester and 15 ml of a 2 M aqueous hydrochloric acid solution was heated at reflux for 4 h. The reaction mixture was cooled to 0 °C and basified by the addition of 3.6 ml of a 32% aqueous solution of sodium hydroxide. Dilution with 10 ml 1,4-dioxane was followed by addition of a solution of 2.42 g (11.1 mmol) di-tert.-butyl dicarbonate in 5 ml 1,4-dioxane. After completed addition the reaction mixture was allowed to slowly warm to room temperature over night. The mixture was extracted methyl tert.-butyl ether at pH 8. The organic layer was extracted with a 1 M aqueous sodium hydroxide solution. The combined aqueous layers were acidified to pH 1 by the addition of and ice cold 2 M aqueous hydrochloric acid solution at 0 °C and extracted with three portions of ethyl acetate. The combined organic layers were dried over sodium sulfate, concentrated in vacuo and purified by flash chromatography to give 1.55 g (91%) of the title compound as an off-white solid.
MS m/e (%): 308 ([M-H⁺]⁻, 100)
[α]_{D} = +31.32 (c = 0.3768, CHCl₃)

### e) (+)-3-(4-Fluoro-phenyl)-4-hydroxymethyl-pyrrolidine-1-carboxylic acid tert-butyl ester

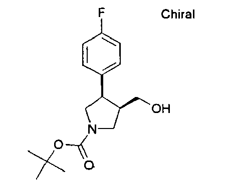

To a solution of 0.75 g (2.4 mmol) (+)-4-(4-fluoro-phenyl)-pyrrolidine-1,3-dicarboxylic acid 1-tert-butyl ester in 12 ml THF were added 2.42 ml (4.84 mmol) of a 2 M borane dimethylsulfide complex solution in THF at 0 °C. The mixture was stirred for 15 min at 0 °C and then for 5 h at room temperature. After cooling to 0 °C the reaction was quenched by the addition of methanol. Stirring was continued until no evolution of gas was observed any more. The reaction mixture was concentrated in vacuo. The residue was purified by flash chromatography to give 0.61 g (85%) of the title compound as a colorless amorphous solid.
MS m/e (%): 296 (M+H⁺, 24)
[α]_{D} = +62.40 (c = 0.423, CHCl₃)

### Intermediate B

### (+)-3-(3-Fluoro-phenyl)-4-hydroxymethyl-pyrrolidine-1-carboxylic acid tert-butyl ester

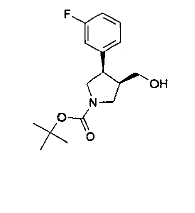

The title compound was obtained as a light yellow viscous oil in comparable yields after flash chromatography according to the procedures described above for the preparation of (+)-3-(4-fluoro-phenyl)-4-hydroxymethyl-pyrrolidine-1-carboxylic acid tert-butyl ester using 3-fluorobenzaldehyde instead of 4-fluorobenzaldehyde in step a).
MS m/e (%): 296 (M+H⁺, 7)
[α]_{D} = +61.86 (c = 0.892, CHCl₃)

### Intermediate C

### (+)-3-(2-Fluoro-phenyl)-4-hydroxymethyl-pyrrolidine-1-carboxylic acid tert-butyl ester

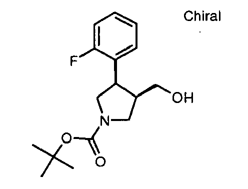

The title compound was obtained as an off-white amorphous solid in comparable yields after flash chromatography according to the procedures described above for the preparation of (+)-3-(4-fluoro-phenyl)-4-hydroxymethyl-pyrrolidine-1-carboxylic acid tert-butyl ester using 2-fluorobenzaldehyde instead of 4-fluorobenzaldehyde in step a). In step c) en an tiomerically pure (+)-1-benzyl-4-(2-fluoro-phenyl)-pyrrolidine-3-carboxylic acid methyl ester was used after separation of the racemic mixture obtained in step b) by preparative HPLC (Chiralcel OD, heptane/EtOH 99:1).
MS m/e (%): 295 (M⁺, 7)
[α]_{D} = +72.54(c = 0.531, CHCl₃)

### Intermediate D

### (+)-3-(4-Chloro-phenyl)-4-hydroxymethyl-pyrrolidine-1-carboxylic acid tert-butyl ester

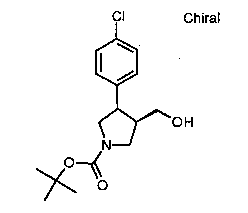

The title compound was obtained as a colorless amorphous solid in comparable yields after flash chromatography according to the procedures described above for the preparation of (+)-3-(4-fluoro-phenyl)-4-hydroxymethyl-pyrrolidine-1-carboxylic acid tert-butyl ester using 4-chlorobenzaldehyde instead of 4-fluorobenzaldehyde in step a).
MS m/e (%): 312 (M+H⁺, 75)
[α]_{D} = +63.80 (c = 0.635, CHCl₃)

### Intermediate E

### (+)-3-(3-Chloro-phenyl)-4-hydroxymethyl-pyrrolidine-1-carboxylic acid tert-butyl ester

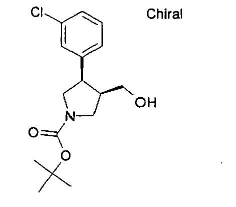

The title compound was obtained as a colorless amorphous solid in comparable yields after flash chromatography according to the procedures described above for the preparation of (+)-3-(4-fluoro-phenyl)-4-hydroxymethyl-pyrrolidine-1-carboxylic acid tert-butyl ester using 3-chlorobenzaldehyde instead of 4-fluorobenzaldehyde in step a). In step c) enantiomericallypure (+)-1-benzyl-4-(3-chloro-phenyl)-pyrrolidine-3-carboxylic acid methyl ester was used after separation of the racemic mixture obtained in step b) by preparative HPLC (Chiralcel OD, heptane/EtOH 98:2).
MS m/e (%): 312 (M+H⁺, 18)
[α]_{D} = +69.19(c = 0.597, CHCl₃)

### Intermediate F

### (+)-3-(2-Chloro-phenyl)-4-hydroxymethyl-pyrrolidine-1-carboxylic acid tert-butyl ester

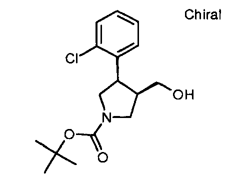

The title compound was obtained as a light yellow amorphous solid in comparable yields after flash chromatography according to the procedures described above for the preparation of (+)-3-(4-fluoro-phenyl)-4-hydroxymethyl-pyrrolidine-1-carboxylic acid tert-butyl ester using 2-chlorobenzaldehyde instead of 4-fluorobenzaldehyde in step a). MS m/e (%): 312 (M+H⁺, 100)
[α]_{D} = +90.85 (c = 0.790, CHCl₃)

### Intermediate G

### (+)-3-Hydroxymethyl-4-o-tolyl-pyrrolidine-1-carboxylic acid tert-butyl ester

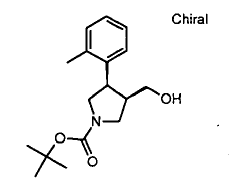

The title compound was obtained as a light yellow amorphous solid in comparable yields after flash chromatography according to the procedures described above for the preparation of (+)-3-(4-fluoro-phenyl)-4-hydroxymethyl-pyrrolidine-1-carboxylic acid tert-butyl ester using o-tolylbenzaldehyde instead of 4-fluorobenzaldehyde in step a).

MS m/e (%): 292 (M+H⁺, 100)
[α]_{D} = +77.97 (c = 1.049, CHCl₃)

### Intermediate H

### (+)-3-Hydroxymethyl-4-phenyl-pyrrolidine-1-carboxylic acid tert-butyl ester

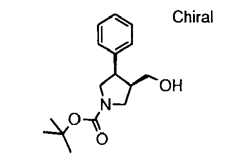

The title compound is obtained as a colorless amorphous solid in comparable yields after flash chromatography according to the procedures described above for the preparation of (+)-3-(4-fluoro-phenyl)-4-hydroxymethyl-pyrrolidine-1-carboxylic acid tert-butyl ester using benzaldehyde instead of 4-fluorobenzaldehyde in step a).
MS m/e (%): 278 (M+H⁺, 10)
[α]_{D} = +71.84 (c = 0.877, CHCl₃)

### Example 1

### (+)-3-(3,5-Bis-trifluoromethyl-benzyloxymethyl)-4-(4-fluoro-phenyl)-pyrrolidine hydrochloride

### a) (+)-3-(3,5-Bis-trifluoromethyl-benzyloxymethyl)-4-(4-fluoro-phenyl)-pyrrolidine-1-carboxylic acid tert-butyl ester

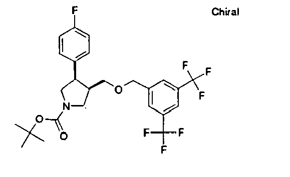

To a solution of 0.17 g (0.58 mmol) (+)-3-(4-fluoro-phenyl)-4-hydroxymethyl-pyrrolidine-1-carboxylic acid tert-butyl ester (Intermediate A) in 6ml DMF were added 0.029 g (0.60 mmol) sodium hydride (50% in mineral oil) at 0 °C. The reaction mixture was allowed to warm to room temperature. After 1 h 0.11 ml (0.60 mmol) 3,5-bis(trifluoromethyl)benzyl bromide were added. Stirring at room temperature for 2.5 h was followed by quenching with 10 ml of water and extraction with three portions of methyl tert.-butylether. The combined organic layers were washed with brine, dried over sodium sulfate and concentrated in vacuo. The crude product was purified by flash column chromatography (silica gel, heptane / ethyl acetate) to give 0.183 g (61%) of the title compound as an amorphous solid.
MS m/e (%): 522 (M+H⁺, 100)
[α]_{D} = +20.20 (c = 1.129, CHCl₃)

### b) (+)-3-(3,5-Bis-trifluoromethyl-benzyloxymethyl)-4-(4-fluoro-phenyl)-pyrrolidine hydrochloride

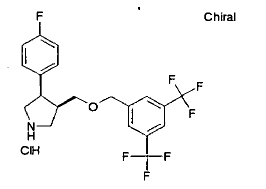

A solution of 0.17 g (0.33 mmol) (+)-3-(3,5-bis-trifluoromethyl-benzyloxymethyl)-4-(4-fluoro-phenyl)-pyrrolidine-1-carboxylic acid tert-butyl ester in 2.65 ml of a 1.25 M solution of hydrochloric acid (3.3 mmol) in methanol was stirred for 30' at 50 °C. The reaction mixture was concentrated to dryness to give 0.14 g (92%) of the title compound as a colorless solid.
MS m/e (%): 422 (M+H⁺, 100)
[α]_{D} = +29.36 (c = 0.504, CHCl₃)

### Example 2

### (+)-3-(3,5-Bis-trifluoromethyl-benzyloxymethyl)-4-(3-fluoro-phenyl)-pyrrolidine hydrochloride

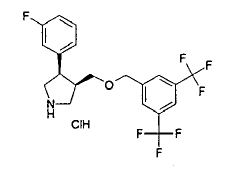

The title compound was obtained as a light yellow solid in comparable yields according to the procedures described above for the preparation of (+)-3-(3,5-bis-trifluoromethyl-benzyloxymethyl)-4-(4-fluoro-phenyl)-pyrrolidine hydrochloride using Intermediate B instead of Intermediate A in step a).
MS m/e (%): 422 (M+H⁺, 100)
[α]_{D} = +31.60 (c = 0.446, CHCl₃)

### Example 3

### (+)-3-(3,5-Bis-trifluoromethyl-benzyloxymethyl)-4-(2-fluoro-phenyl)-pyrrolidine hydrochloride

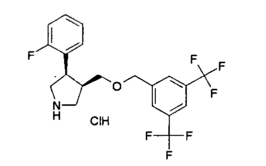

The title compound was obtained as a light yellow solid in comparable yields according to the procedures described above for the preparation of (+)-3-(3,5-bis-trifluoromethyl-benzyloxymethyl)-4-(4-fluoro-phenyl)-pyrrolidine hydrochloride using Intermediate C instead of Intermediate A in step a).
MS m/e (%): 422 (M+H⁺, 100)
[α]_{D} = +40.56 (c = 0.385, CHCl₃)

### Example 4

### (+)-3-(3,5-Bis-trifluoromethyl-benzyloxymethyl)-4-(4-chloro-phenyl)-pyrrolidine hydrochloride

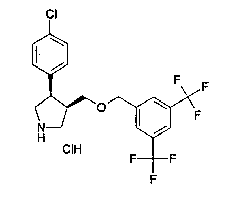

The title compound was obtained as a light yellow solid in comparable yields according to the procedures described above for the preparation of (+)-3-(3,5-bis-trifluoromethyl-benzyloxymethyl)-4-(4-fluoro-phenyl)-pyrrolidine hydrochloride using Intermediate D instead of Intermediate A in step a).
MS m/e (%): 438 (M+H⁺, 100)
[α]_{D} = +29.97 (c = 0.394, CHCl₃)

### Example 5

### (+)-3-(3,5-Bis-trifluoromethyl-benzyloxymethyl)-4-(3-chloro-phenyl)-pyrrolidine hydrochloride

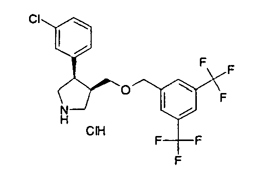

The title compound was obtained as a light yellow solid in comparable yields according to the procedures described above for the preparation of (+)-3-(3,5-bis-trifluoromethyl-benzyloxymethyl)-4-(4-fluoro-phenyl)-pyrrolidine hydrochloride using Intermediate E instead of Intermediate A in step a).
MS m/e (%): 438 (M+H⁺, 100)
[α]_{D} = +35.06 (c = 0.685, CHCl₃)

### Example 6

### (+)-3-(3,5-Bis-trifluoromethyl-benzyloxymethyl)-4-(2-chloro-phenyl)-pyrrolidine hydrochloride

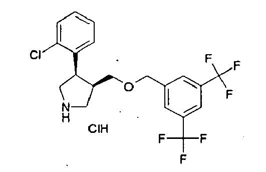

The title compound was obtained as a light yellow solid in comparable yields according to the procedures described above for the preparation of (+)-3-(3,5-bis-trifluoromethyl-benzyloxymethyl)-4-(4-fluoro-phenyl)-pyrrolidine hydrochloride using Intermediate F instead of Intermediate A in step a).
MS m/e (%): 438 (M+H⁺, 100)
[α]_{D} = +53.72 (c = 0.672, CHCl₃)

### Example 7

### (+)-3-(3,5-Bis-trifluoromethyl-benzyloxymethyl)-4-o-tolyl-pyrrolidine hydrochloride

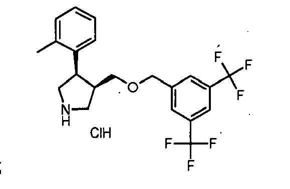

The title compound was obtained as a light yellow solid in comparable yields according to the procedures described above for the preparation of (+)-3-(3,5-bis-trifluoromethyl-benzyloxymethyl)-4-(4-fluoro-phenyl)-pyrrolidine hydrochloride using Intermediate G instead of Intermediate A in step a).
MS m/e (%): 418 (M+H⁺, 100)
[α]_{D} = +47.53 (c = 0.962, CHCl₃)

### Example 8

### (+)-3-(3,5-Bis-trifluoromethyl-benzyloxymethyl)-4-phenyl-pyrrolidine hydrochloride

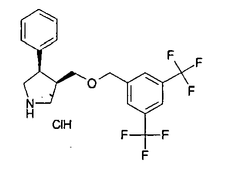

The title compound was obtained as a light yellow solid in comparable yields according to the procedures described above for the preparation of (+)-3-(3,5-bis-trifluoromethyl-benzyloxymethyl)-4-(4-fluoro-phenyl)-pyrrolidine hydrochloride using Intermediate H instead of Intermediate A in step a).
MS m/e (%): 404 (M+H⁺, 100)
[α]_{D} = +22.79 (c = 0.592, CHCl₃)

### Example 9

### (+)-3-(3,5-Dibromo-benzyloxymethyl)-4-phenyl-pyrrolidine hydrochloride

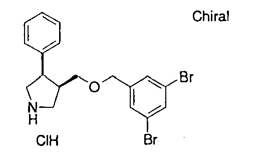

The title compound was obtained as an off-white solid in comparable yields according to the procedures described above for the preparation of (+)-3-(3,5-bis-trifluoromethyl-benzyloxymethyl)-4-(4-fluoro-phenyl)-pyrrolidine hydrochloride using Intermediate H instead of Intermediate A in step a).
MS m/e (%): 426 (M+H⁺, 100)
[α]_{D} = +39.42 (c = 0.416, CHCl₃)

### Example 10

### (+)-3-(3,5-Dimethyl-benzyloxymethyl)-4-phenyl-pyrrolidine hydrochloride

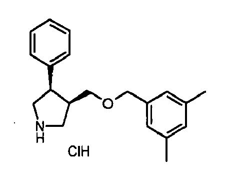

The title compound was obtained as an off-white solid in comparable yields according to the procedures described above for the preparation of (+)-3-(3,5-bis-trifluoromethyl-benzyloxymethyl)-4-(4-fluoro-phenyl)-pyrrolidine hydrochloride using Intermediate H instead of Intermediate A and 3,5-dimethylbenzyl bromide instead of 3,5-bis(trifluoromethyl)benzyl bromide in step a).
MS m/e (%): 296 (M+H⁺, 100)
[α]_{D} = +58.67 (c = 0.375, CHCl₃)

### Example 11

### (+)-3-(3-Fluoro-5-trifluoromethyl-benzyloxymethyl)-4-phenyl-pyrrolidine hydrochloride

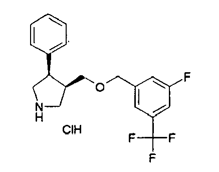

The title compound was obtained as an off-white solid in comparable yields according to the procedures described above for the preparation of (+)-3-(3,5-bis-trifluoromethyl-benzyloxymethyl)-4-(4-fluoro-phenyl)-pyrrolidine hydrochloride using Intermediate H instead of Intermediate A and 3-fluoro-5-(trifluoromethyl)benzyl bromide instead of 3,5-bis(trifluoromethyl)benzyl bromide in step a).
MS m/e (%): 354 (M+H⁺, 100)
[α]_{D} = +41.40 (c = 0.722, CHCl₃)

### Example 12

### (+)-3-Phenyl-4-(3-trifluoromethyl-benzyloxymethyl)-pyrrolidine hydrochloride

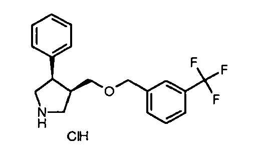

The title compound was obtained as an off-white solid in comparable yields according to the procedures described above for the preparation of (±)-3-(3,5-bis-trifluoromethyl-benzyloxymethyl)-4-(4-fluoro-phenyl)-pyrrolidine hydrochloride using Intermediate H instead of Intermediate A and 3-(trifluoromethyl)benzyl bromide instead of 3,5-bis(trifluoromethyl)benzyl bromide in step a).
MS m/e (%): 336 (M+H⁺, 100)
[α]_{D} = +41.84 (c = 0.619, CHCl₃)

### Example 13

### (+)-3-Phenyl-4-(3-trifluoromethoxy-benzyloxymethyl)-pyrrolidine hydrochloride

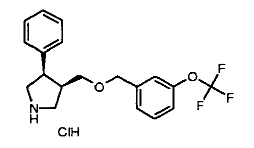

The title compound was obtained as an off-white solid in comparable yields according to the procedures described above for the preparation of (+)-3-(3,5-bis-trifluoromethyl-benzyloxymethyl)-4-(4-fluoro-phenyl)-pyrrolidine hydrochloride using Intermediate H instead of Intermediate A and 3-(trifluoromethoxy)benzyl bromide instead of 3,5-bis(trifluoromethyl)benzyl bromide in step a).
MS m/e (%): 352 (M+H⁺, 100)
[α]_{D} = +42.31 (c = 0.643, CHCl₃)

### Example 14

### (+)-3-(3,5-Dimethoxy-benzyloxymethyl)-4-phenyl-pyrrolidine hydrochloride

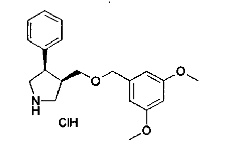

The title compound was obtained as an off-white solid in comparable yields according to the procedures described above for the preparation of (+)-3-(3,5-bis-trifluoromethyl-benzyloxymethyl)-4-(4-fluoro-phenyl)-pyrrolidine hydrochloride using Intermediate H instead of Intermediate A and 3,5-(dimethoxy)benzyl bromide instead of 3,5-bis(trifluoromethyl)benzyl bromide in step a).
MS m/e (%): 328 (M+H⁺, 100)
[α]_{D} = +43.53 (c = 0.611, CHCl₃)

### Example 15

### (+)-3-Phenyl-4-(2-trifluoromethyl-benzyloxymethyl)-pyrrolidine hydrochloride

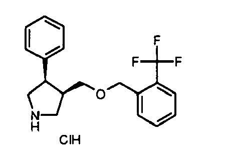

The title compound was obtained as an off-white solid in comparable yields according to the procedures described above for the preparation of (+)-3-(3,5-bis-trifluoromethyl-benzyloxymethyl)-4-(4-fluoro-phenyl)-pyrrolidine hydrochloride using Intermediate H instead of Intermediate A and 2-(trifluoromethyl)benzyl bromide instead of 3,5-bis(trifluoromethyl)benzyl bromide in step a).
MS m/e (%): 336 (M+H⁺, 100)
[α]_{D} = +40.40 (c = 0.654, CHCl₃)

### Example 16

### (+)-(3,5-Bis-trifluoromethyl-benzyl)-((3R,4R)-4-phenyl-pyrrolidin-3-ylmethyl)-amine dihydrochloride

### a) (+)-(3R,4R)-3-Aminomethyl-4-phenyl-pyrrolidine-1-carboxylic acid tert-butyl ester

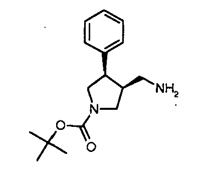

To a solution of 2.00 g (7.21 mmol) (+)-(3R,4R)-3-hydroxymethyl-4-phenyl-pyrrolidine-1-carboxylic acid tert-butyl ester (Intermediate H) and 1.11 ml (7.94 mmol) triethylamine in 35 ml dichloromethane were added dropwise at 0 °C 0.59 ml (7.6 mmol) methanesulfonyl chloride. After completed addition the reaction mixture was allowed to warm to room temperature during 30 minutes. Quenching with water was followed by extraction with two portions of methyl tert.-butyl ether. The combined organic layers were washed with water and brine, dried over sodium sulfate and concentrated in vacuo to give 2.57 g (100%) of crude (3R,4R)-3-methanesulfonyloxymethyl-4-phenylpyrrolidine-1-carboxylic acid tert-butyl ester, which was used in the next step without purification.

A mixture of 1.30 g (3.66 mmol) crude (3R,4R)-3-methanesulfonyloxymethyl-4-phenylpyrrolidine-1-carboxylic acid tert-butyl ester and 357 mg (5.49 mmol) sodium azide in 8 ml dimethylsulfoxide was heated at 120 °C for 5 h. The mixture was cooled to room temperature, diluted with a 0.2 M aqueous solution of sodium carbonate and extracted with two portions of methyl tert.-butyl ether. The combined organic layers were washed with water and brine, dried over sodium sulfate and concentrated in vacuo to give 1.07 g (97%) of crude (3R,4R)-3-azidomethyl-4-phenyl-pyrrolidine-1-carboxylic acid tert-butyl ester This material was dissolved in 17.5 ml THF and treated with 975 mg (3.71 mmol) triphenylphosphine and 1.2 ml of water. After stirring at room temperature for 20 h the reaction mixture was diluted with methyl tert.-butyl ether and washed with three portions of a half-saturated aqueous solution of ammonium chloride. The combined aqueous layers were basified by the addition of 2 M aqueous sodium hydroxide solution and extracted with three portions of methyl tert.-butyl ether. The combined organic layers were dried over sodium sulfate and concentrated in vacuo to give 0.74 g (75%) of the crude title compound as a colorless amorphous solid.
MS m/e (%) :277 (M+H⁺, 21)
[α]_{D} = +52.30 (c = 0.579, CHCl₃)

### b) (3S,4R)-3-[(3,5-Bis-trifluoromethyl-benzoylamino)-methyl]-4-phenyl-prrolidine-1-carboxylic acid tert-butyl ester

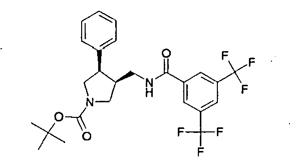

To a solution of 0.36 g (1.3 mmol) (+)-(3R,4R)-3-aminomethyl-4-phenyl-pyrrolidine-1-carboxylic acid tert-butyl ester and 0.22 ml (1.6 mmol) triethylamine in 6.5 ml dichloromethane were added 0.26 ml (1.4 mmol) 3,5-bis(trifluoromethyl)benzoyl chloride at 0 °C. After completed addition the reaction mixture was allowed to warm to room temperature and stirred for one hour. Quenching with water was followed by basification with 1 M aqueous sodium hydroxide solution and extraction with three portions of methyl tert.-butyl ether. The combined organic layers were washed with brine, dried over sodium sulfate and concentrated in vacuo. Flash column chromatography gave 0.62 g (92%) of the title compound as a white solid.
MS m/e (%) : 517 (M+H⁺, 21)

### c)-(+)-(3,5-Bis-trifluoromethyl-benzyl)-((3R,4R)-4-phenyl-pyrrolidin-3-ylmethyl)-amine dihydrochloride

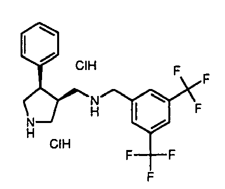

A mixture of 0.29 g (0.57 mmol) (3S,4R)-3-[(3,5-bis-trifluoromethyl-benzoylamino)-methyl]-4-phenyl-pyrrolidine-1-carboxylic acid tert-butyl ester and 0.28 ml of a 2 M THF solution of borane dimethylsulide complex (0.56 mmol) in 6 ml toluene was heated at reflux for 20 h. After addition of another portion of 0.28 ml of a 2 M THF solution of borane dimethylsulfide complex (0.56 mmol) the mixture was heated at reflux for one more hour. After cooling to room temperature 1 ml of methanol was added, and the mixture was heated to reflux for 30 minutes. Concentration in vacuo was followed by flash column chromatography to give the free base of the title compound. Dissolution in a 1.25 M solution of hydrochloric acid and concentration in vacuo gave 91 mg (34%) of the title compound as a light yellow solid.
MS m/e (%): 403 (M+H⁺, 100)
[α]_{D} = +12.60 (c = 0.500, MeOH)

### Example 17

### (3,5-Bis-trifluoromethyl-benzyl)-methyl-((3R,4R)-4-phenyl-pyrrolidin-3-ylmethyl)-amine dihydrochloride

### a) (3S,4R)-3-{[(3,5-Bis-trifluoromethyl-benzoyl)-methyl-amino]-methyl}-4-phenylpyrrolidine-1-carboxylic acid tert-butyl ester

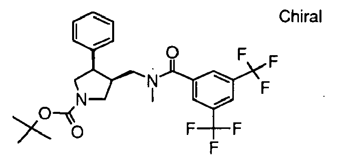

To a solution of 0.20 g (0.39 mmol) (3S,4R)-3-[(3,5-bis-trifluoromethyl-benzoylamino)-methyl]-4-phenyl-pyrrolidine-1-carboxylic acid tert-butyl ester (Example 20 of I b)) in 4 ml DMF were added 20 mg (0.42 mmol) sodium hydride (50% in mineral oil) at room temperature. After 30 minutes 0.027 ml (0.42 mmol) methyl iodide were added, and stirring was continued for 2 h. Quenching with water was followed by extraction with two portions of methyl tert.-butyl ether. The combined organic layers were washed with water and brine, dried over sodium sulfate and concentrated in vacuo. Flash column chromatography gave 0.19 g (93%) of the title compound as a white solid.

MS m/e (%) :531 (M+H⁺, 22)

### b) (3,5-Bis-trifluoromethyl-benzyl)-methyl-((3R,4R)-4-phenyl-pyrrolidin-3-ylmethyl)-amine dihydrochloride

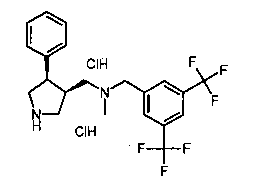

A mixture of 0.12 g (0.23 mmol) (3S,4R)-3-{[(3,5-bis-trifluoromethyl-benzoyl)-methyl-amino]-methyl}-4-phenyl-pyrrolidine-1-carboxylic acid tert-butyl ester and 0.11 ml of a 2 M THF solution of borane dimethylsulide complex (0.22 mmol) in 2 ml toluene was heated at reflux for 20 h. After cooling to room temperature a 2 M aqueous hydrochloric acid solution was added, and the mixture was heated to reflux for 30 minutes. The reaction mixture was cooled to room temperature, diluted with 1 M aqueous hydrochloric acid solution and washed with two portions of methyl tert.-butyl ether. The combined organic layers were extracted with 1 M aqueous hydrochloric acid solution. The combined aqueous layers were basified with 2 M aqueous sodium hydroxide solution and extracted with three portions of dichloromethane. The combined organic extracts were dried over sodium sulfate and concentrated in vacuo. Flash column chromatography gave the free base of the title compound. This material was purified further by trituration with warm methyl tert.-butyl ether followed by filtration and concentration of the filtrate in vacuo. Dissolution in a 1.25 M solution of hydrochloric acid and concentration in vacuo gave 55 mg (50%) of the title compound as a white solid. MS m/e (%) : 417 (M+H⁺, 100)

## Claims

1. Cis-derivatives of formula 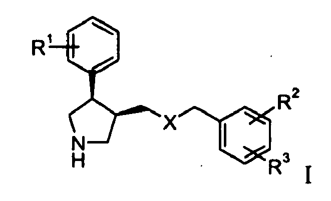 wherein
R¹ is hydrogen, halogen or C₁₋₇-alkyl;
R²/R³ are independently from each other hydrogen, halogen, CN, C₁₋₇-alkyl substituted by halogen or C₁₋₇-alkoxy substituted by halogen;
X is -O- or -N(R)-;
R is hydrogen or C₁₋₇-alkyl;
and pharmaceutically acceptable acid addition salts thereof.

2. Compounds of formula 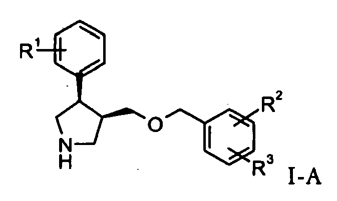 according to claim 1,
wherein
R¹ is hydrogen, halogen or C₁₋₇-alkyl;
R²/R³ are independently from each other hydrogen, halogen, CN, C₁₋₇-alkyl substituted by halogen or C₁₋₇-alkoxy substituted by halogen;
and pharmaceutically acceptable acid addition salts thereof.

3. Compounds of formula 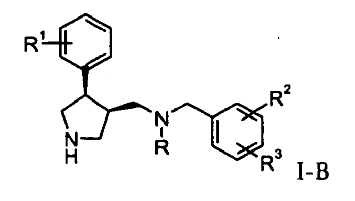 according to claim 1,
wherein
R¹ is hydrogen, halogen or C₁₋₇-alkyl;
R²/R³ are independently from each other hydrogen, halogen, CN, C₁₋₇-alkyl substituted by halogen or C₁₋₇-alkoxy substituted by halogen;
R is hydrogen or C₁₋₇-alkyl;
and pharmaceutically acceptable acid addition salts thereof.

4. Compounds of formula I-A according to claim 2, which compounds are
(+)-(3R,4R)-3-(3,5-bis-trifluoromethyl-benzyloxymethyl)-4-(4-fluoro-phenyl)-pyrrolidine,
(+)-(3R,4R)-3-(3,5-bis-trifluoromethyl-benzyloxymethyl)-4-(3-fluoro-phenyl)-pyrrolidine,
(+)-(3R,4R)-3-(3,5-bis-trifluoromethyl-benzyloxymethyl)-4-(2-fluoro-phenyl)-pyrrolidone,
(+)-(3R,4R)-3-(3,5-bis-trifluoromethyl-benzyloxymethyl)-4-(4-chloro-phenyl)-pyrrolidine,
(+)-(3R,4R)-3-(3,5-bis-trifluoromethyl-benzyloxymethyl)-4-(3-chloro-phenyl)-pyrrolidine,
(+)-(3R,4R)-3-(3,5-bis-trifluoromethyl-benzyloxymethyl)-4-(2-chloro-phenyl)-pyrrolidine,
(+)-(3R,4R)-3-(3,5-bis-trifluoromethyl-benzyloxymethyl)-4-o-tolyl-pyrrolidine,
(+)-(3R,4R)-3-(3,5-bis-trifluoromethyl-benzyloxymethyl)-4-phenyl-pyrrolidine,
3-(3R,4R)- (3,5-dibromo-benzyloxymethyl)-4-phenyl-pyrrolidine,
(+)-(3R,4R)-3-(3,5-dimethyl-benzyloxymethyl)-4-phenyl-pyrrolidine,
(+)-(3R,4R)-3-(3-fluoro-5-trifluoromethyl-benzyloxymethyl)-4-phenyl-pyrrolidine,
(+)-(3R,4R)-3-phenyl-4-(3-trifluoromethyl-benzyloxymethyl)-pyrrolidine,
(+)-(3R,4R)-3-phenyl-4-(3-trifluoromethoxy-benzyloxymethyl)-pyrrolidine,
(+)-(3R,4R)-3-(3,5-dimethoxy-benzyloxymethyl)-4-phenyl-pyrrolidine,
(+)-(3R,4R)-3-phenyl-4-(2-trifluoromethyl-benzyloxymethyl)-pyrrolidine.

5. Compounds of formula I-B according to claim 3, which compounds are
(+)-(3,5-bis-trifluoromethyl-benzyl)-((3R,4R)-4-phenyl-pyrrolidin-3-ylmethyl)-amine dihydrochloride or
(3,5-bis-trifluoromethyl-benzyl)-methyl-((3R,4R)-4-phenyl-pyrrolidin-3-ylmethyl)-amine dihydrochloride.

6. Process for preparation of compounds of formula I and their pharmaceutically acceptable salts, which process comprises
b) reacting a compound of formula 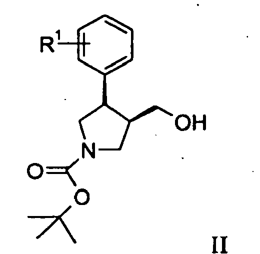 with sodium hydride and a compound of formula 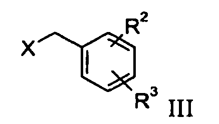 followed by treatment with an acid, selected from HCl or trifluoroacetic acid to give a compound of formula 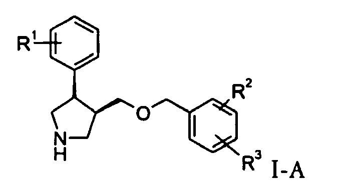 wherein X is Cl, Br or I and R¹ to R³ are as described in claim 1, or
b) reacting a compound of formula 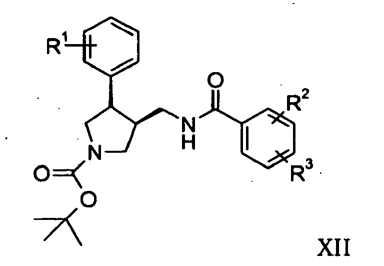 with a THF solution of borane dimethylsulfide complex in toluene and then with an acid selected from HCl or trifluoroacetic acid to a compound of formula 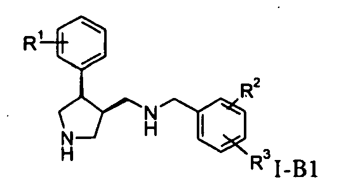 wherein the substituents are as described in claim 1, or
c) alkylating a compound of formula 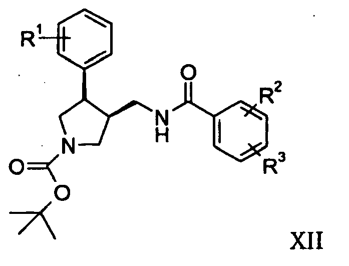 with the alkylating agent methyliodide to a compound of formula 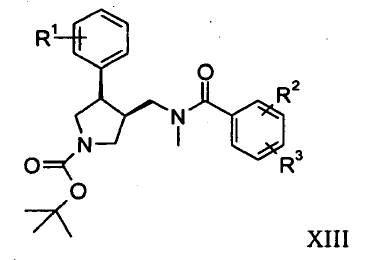 which is consecutively reacted with a THF solution ofborane dimethylsulfide complex in toluene and then with an acid selected from HCl or trifluoroacetic acid to a compound of formula 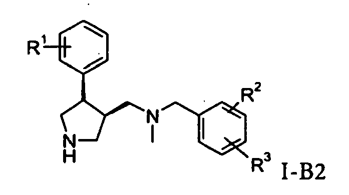
wherein the substituents are as described in claim 1, or
if desired, converting the compounds obtained into pharmaceutically acceptable acid addition salts.

7. A medicament containing one or more compounds as claimed in claim 1 and pharmaceutically acceptable excipients for the treatment of anxiety and depression.

8. The use of a compound as claimed in claim 1 for the manufacture of medicaments for the treatment of anxiety and depression.

## Patentansprüche

1. Cis-Derivate der Formel 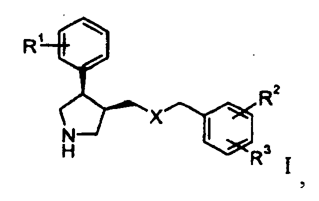 worin
R¹ Wasserstoff, Halogen oder C₁₋₇-Alkyl ist;
R²/R³ unabhängig voneinander Wasserstoff, Halogen, CN, C₁₋₇-Alkyl, substituiert durch Halogen, oder C₁₋₇-Alkoxy, substituiert durch Halogen, sind;
X -O- oder -N(R)- ist;
R Wasserstoff oder C₁₋₇-Alkyl ist;
und pharmazeutisch akzeptable Säureadditionssalze davon.

2. Verbindungen der Formel 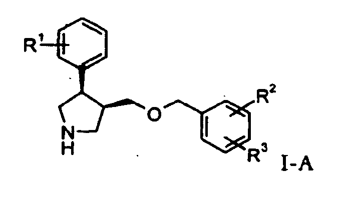 nach Anspruch 1,
worin
R¹ Wasserstoff, Halogen oder C₁₋₇-Alkyl ist;
R²/R³ unabhängig voneinander Wasserstoff, Halogen, CN, C₁₋₇-Alkyl, substituiert durch Halogen, oder C₁₋₇-Alkoxy, substituiert durch Halogen, sind;
und pharmazeutisch akzeptable Säureadditionssalze davon.

3. Verbindungen der Formel 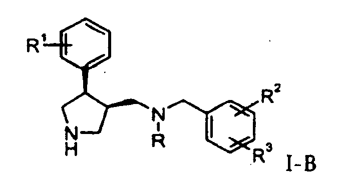 nach Anspruch 1,
worin
R¹ Wasserstoff, Halogen oder C₁₋₇-Alkyl ist;
R²/R³ unabhängig voneinander Wasserstoff, Halogen, CN, C₁₋₇-Alkyl, substituiert durch Halogen, oder C₁₋₇-Alkoxy, substituiert durch Halogen, sind;
R Wasserstoff oder C₁₋₇-Alkyl ist;
und pharmazeutisch akzeptable Säureadditionssalze davon.

4. Verbindungen der Formel I-A nach Anspruch 2, wobei die Verbindungen
(+)-(3R,4R)-3-(3,5-Bis-trifluormethyl-benzyloxymethyl)-4-(4-fluor-phenyl)-pyrrolidin,
(+)-(3R,4R)-3-(3,5-Bis-trifluormethyl-benzyloxymethyl)-4-(3-fluor-phenyl)-pyrrolidin,
(+)-(3R,4R)-3-(3,5-Bis-trifluormethyl-benzyloxymethyl)-4-(2-fluor-phenyl)-pyrrolidin,
(+)-(3R,4R)-3-(3,5-Bis-trifluormethyl-benzyloxymethyl)-4-(4-chlor-phenyl)-pyrrolidin,
(+)-(3R,4R)-3-(3,5-Bis-trifluormethyl-benzyloxymethyl)-4-(3-chlor-phenyl)-pyrrolidin,
(+)-(3R,4R)-3-(3,5-Bis-trifluormethyl-benzyloxymethyl)-4-(2-chlor-phenyl)-pyrrolidin,
(+)-(3R,4R)-3-(3,5-Bis-trifluormethyl-benzyloxymethyl)-4-o-tolyl-pyrrolidin,
(+)-(3R,4R)-3-(3,5-Bis-trifluormethyl-benzyloxymethyl)-4-phenyl-pyrrolidin,
3-(3R,4R)-(3,5-Dibrom-benzyloxymethyl)-4-phenyl-pyrrolidin,
(+)-(3R,4R)-3-(3,5-Dimethyl-benzyloxymethyl)-4-phenyl-pyrrolidin,
(+)-(3R,4R)-3-(3-Fluor-5-trifluormethyl-benzyloxymethyl)-4-phenyl-pyrrolidin,
(+)-(3R,4R)-3-Phenyl-4-(3-trifluormethyl-benzyloxymethyl)-pyrrolidin,
(+)-(3R,4R)-3-Phenyl-4-(3-trifluormethoxy-benzyloxymethyl)-pyrrolidin,
(+)-(3R,4R)-3-(3,5-Dimethoxy-benzyloxymethyl)-4-phenyl-pyrrolidin,
(+)-(3R,4R)-3-Phenyl-4-(2-trifluormethyl-benzyloxymethyl)-pyrrolidin sind.

5. Verbindungen der Formel I-B nach Anspruch 3, wobei die Verbindungen
(+)-(3,5-Bis-trifluormethyl-benzyl)-((3R,4R)-4-phenyl-pyrrolidin-3-ylmethyl)-amin-dihydrochlorid oder
(3,5-Bis-trifluormethyl-benzyl)-methyl-((3R,4R)-4-phenyl-pyrrolidin-3-ylmethyl)-amin-dihydrochlorid sind.

6. Verfahren zur Herstellung von Verbindungen der Formel I und deren pharmazeutisch akzeptablen Salzen, wobei das Verfahren
a) das Umsetzen einer Verbindung der Formel 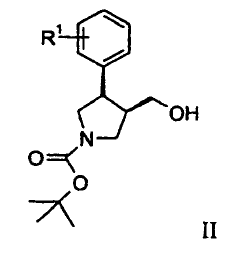 mit Natriumhydrid und einer Verbindung der Formel 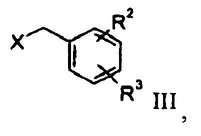 gefolgt von der Behandlung mit einer Säure, ausgewählt aus HCl oder Trifluoressigsäure, unter Erhalt einer Verbindung der Formel 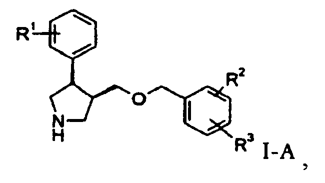 worin X Cl, Br oder I ist und R¹ bis R³ wie in Anspruch 1 beschrieben sind, oder
b) das Umsetzen einer Verbindung der Formel 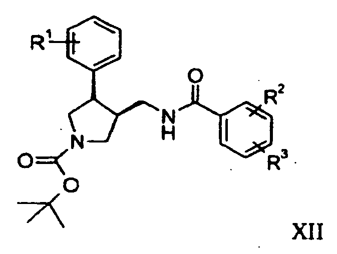 mit einer THF-Lösung von Borandimethylsulfidkomplex in Toluol und dann mit einer Säure, ausgewählt aus HCl oder Trifluoressigsäure, zu einer Verbindung der Formel 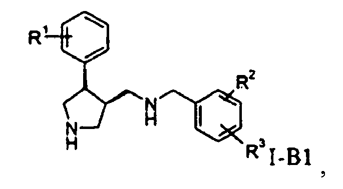 worin die Substituenten wie in Anspruch 1 beschrieben sind, oder
c) das Alkylieren einer Verbindung der Formel 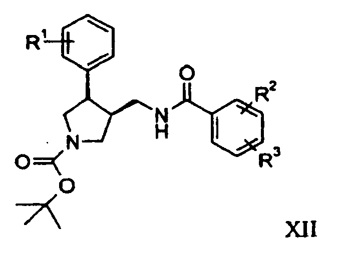 mit dem Alkylierungsmittel Methyliodid zu einer Verbindung der Formel 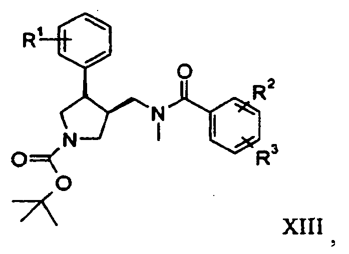 welche nacheinander mit einer THF-Lösung von Borandimethylsulfidkomplex in Toluol und dann mit einer Säure, ausgewählt aus HCl oder Trifluoressigsäure, zu einer Verbindung der Formel 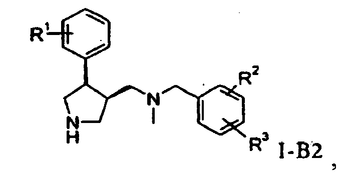
worin die Substituenten wie in Anspruch 1 beschrieben sind, umgesetzt wird, oder, wenn gewünscht, das Umwandeln der erhaltenen Verbindungen in pharmazeutisch akzeptable Säureadditionssalze umfasst.

7. Medikament, enthaltend eine oder mehrere Verbindungen nach Anspruch 1 und pharmazeutisch akzeptable Hilfsstoffe, zur Behandlung von Angst und Depression.

8. Verwendung einer Verbindung nach Anspruch 1 für die Herstellung von Medikamenten zur Behandlung von Angst und Depression.

## Revendications

1. Dérivés cis de formule 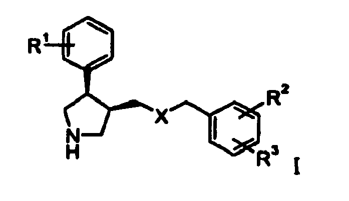 dans laquelle
R¹ est un hydrogène, un halogène ou un alkyle en C₁-C₇;
R²/R³ sont indépendamment l'un de l'autre un hydrogène, un halogène, CN, un alkyle en C₁-C₇ substitué par un halogène ou un alcoxy en C₁-C₇ substitué par un halogène;
X est -O- ou -N(R)-;
R est un hydrogène ou un alkyle en C₁-C₇;
et leurs sels d'addition d'acides pharmaceutiquement acceptables.

2. Composés de formule 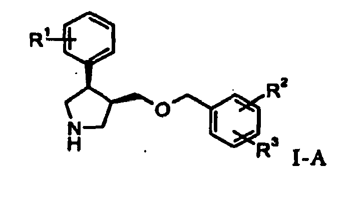 selon la revendication 1, dans lesquels
R¹ est un hydrogène, un halogène ou un alkyle en C₁-C₇;
R²/R³ sont indépendamment l'un de l'autre un hydrogène, un halogène, CN, un alkyle en C₁-C₇ substitué par un halogène ou un alcoxy en C₁-C₇ substitué par un halogène;
et leurs sels d'addition d'acides pharmaceutiquement acceptables.

3. Composés de formule 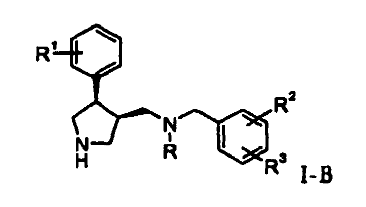 selon la revendication 1, dans lesquels
R¹ est un hydrogène, un halogène ou un alkyle en C₁-C₇;
R²/R³ sont indépendamment l'un de l'autre un hydrogène, un halogène, CN, un alkyle en C₁-C₇ substitué par un halogène ou un alcoxy en C₁-C₇ substitué par un halogène;
R est un hydrogène ou un alkyle en C₁-C₇;
et leurs sels d'addition d'acides pharmaceutiquement acceptables.

4. Composés de formule I-A selon la revendication 2, ces composés étant:
la (+)-(3R,4R)-3-(3,5-bis-trifluorométhylbenzyloxyméthyl)-4-(4-fluorophényl)-pyrrolidine,
la (+)-(3R,4R)-3-(3,5-bis-trifluorométhylbenzyloxyméthyl)-4-(3-fluorophényl)-pyrrolidine,
la (+)-(3R,4R)-3-(3,5-bis-trifluorométhylbenzyloxyméthyl)-4-(2-fluorophényl)-pyrrolidine,
la (+)-(3R,4R)-3-(3,5-bis-trifluorométhylbenzyloxyméthyl)-4-(4-chlorophényl)-pyrrolidine,
la (+)-(3R,4R)-3-(3,5-bis-trifluorométhylbenzyloxyméthyl)-4-(3-chlorophényl)-pyrrolidine,
la (+)-(3R,4R)-3-(3,5-bis-trifluorométhylbenzyloxyméthyl)-4-(2-chlorophényl)-pyrrolidine,
la (+)-(3R,4R)-3-(3,5-bis-trifluorométhylbenzyloxyméthyl)-4-o-tolylpyrrolidine,
la (+)-(3R,4R)-3-(3,5-bis-trifluorométhylbenzyloxyméthyl)-4-phénylpyrrolidine,
la 3-(3R,4R)-(3,5-dibromobenzyloxyméthyl)-4-phénylpyrrolidine,
la (+)-(3R,4R)-3-(3,5-diméthylbenzyloxyméthyl)-4-phénylpyrrolidine,
la (+)-(3R,4R)-3-(3-fluoro-5-trifluorométhylbenzyloxyméthyl)-4-phénylpyrrolidine,
la (+)-(3R,4R)-3-phényl-4-(3-trifluorométhylbenzyloxyméthyl)pyrrolidine,
la (+)-(3R,4R)-3-phényl-4-(3-trifluorométhoxybenzyloxyméthyl)pyrrolidine,
la (+)-(3R,4R)-3-(3,5-diméthoxybenzyloxyméthyl)-4-phénylpyrrolidine,
la (+)-(3R,4R)-3-phényl-4-(2-trifluorométhylbenzyloxyméthyl)pyrrolidine.

5. Composés de formule I-B selon la revendication 3, ces composés étant:
le dichlorhydrate de (+)-(3,5-bis-trifluorométhylbenzyl)-((3R,4R)-4-phényl-pyrrolidin-3-ylméthyl)amine ou
le dichlorhydrate de (3,5-bis-trifluorométhylbenzyl)méthyl-((3R,4R)-4-phényl-pyrrolidin-3-ylméthyl)amine.

6. Procédé de préparation de composés de formule I et de leurs sels pharmaceutiquement acceptables, ce procédé comprenant:
a) la réaction d'un composé de formule 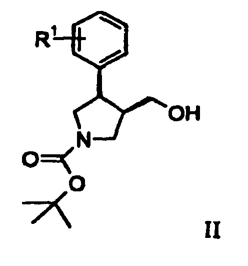 avec de l'hydrure de sodium et un composé de formule 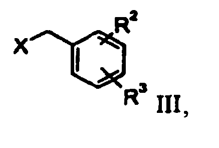 suivie d'un traitement avec un acide choisi parmi HCl et l'acide trifluoroacétique, pour obtenir un composé de formule 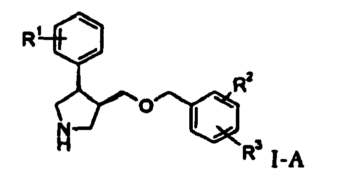 où X est Cl, Br ou I et R¹ à R³ sont tels que décrits dans la revendication 1, ou
b) la réaction d'un composé de formule 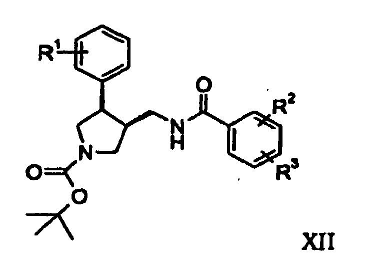 avec une solution dans du THF de complexe de borane-sulfure de diméthyle dans du toluène, puis avec un acide choisi parmi HCl et l'acide trifluoroacétique, pour obtenir un composé de formule 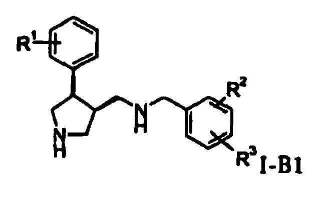 où les substituants sont tels que décrits dans la revendication 1, ou
c) l'alkylation d'un composé de formule 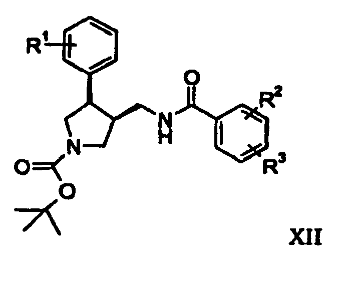 avec l'agent d'alkylation iodure de méthyle pour obtenir un composé de formule 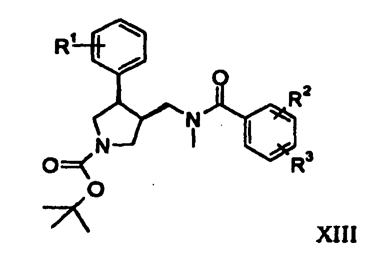 que l'on fait ensuite réagir avec une solution dans du THF de complexe de borane-sulfure de diméthyle dans du toluène, puis avec un acide choisi parmi HCl et l'acide trifluoroacétique pour obtenir un composé de formule 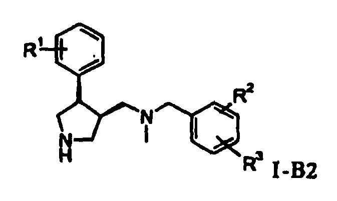
où les substituants sont tels que décrits dans la revendication 1, ou
si désiré, la conversion des composés obtenus en sels d'addition d'acides pharmaceutiquement acceptables.

7. Médicament contenant un ou plusieurs composés selon la revendication 1 et des excipients pharmaceutiquement acceptables pour le traitement de l'anxiété et de la dépression.

8. Utilisation d'un composé selon la revendication 1 pour la fabrication de médicaments destinés au traitement de l'anxiété et de la dépression.
